(19) Europäisches Patentamt
European Patent Office
Office européen des brevets

(11) **EP 3 390 693 B1**

(12) **EUROPÄISCHE PATENTSCHRIFT**

(45) Veröffentlichungstag und Bekanntmachung des Hinweises auf die Patenterteilung:
**27.01.2021 Patentblatt 2021/04**

(21) Anmeldenummer: **16820204.2**

(22) Anmeldetag: **14.12.2016**

(51) Int Cl.:
*C25B 1/00* (2021.01)   *C25B 1/04* (2021.01)
*C25B 3/04* (2006.01)   *C25B 15/08* (2006.01)
*C07C 29/151* (2006.01)   *C07C 41/01* (2006.01)
*C10L 3/08* (2006.01)   *C10G 2/00* (2006.01)

(86) Internationale Anmeldenummer:
**PCT/EP2016/080942**

(87) Internationale Veröffentlichungsnummer:
**WO 2017/102817 (22.06.2017 Gazette 2017/25)**

(54) **VERFAHREN ZUR ERZEUGUNG VON KOHLENSTOFF-BASIERTEN SEKUNDÄRENERGIETRÄGERN ODER BASISCHEMIKALIEN**

METHOD FOR GENERATING CARBON-BASED SECONDARY ENERGY CARRIERS OR BASIC CHEMICAL PRODUCTS

PROCÉDÉ DE PRODUCTION DE SUPPORTS D'ÉNERGIE SECONDAIRE À BASE DE CARBONE OU DE PRODUITS CHIMIQUES DE BASE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2015   DE 102015226111**

(43) Veröffentlichungstag der Anmeldung:
**24.10.2018   Patentblatt 2018/43**

(73) Patentinhaber: **Zentrum für Sonnenenergie- und Wasserstoff-Forschung Baden-Württemberg Gemeinnützige Stiftung**
**70563 Stuttgart (DE)**

(72) Erfinder:
• **BRELLOCHS, Jochen**
**70569 Stuttgart (DE)**
• **LÖFFLER, Marc-Simon**
**70199 Stuttgart (DE)**
• **SPECHT, Michael**
**71111 Waldenbuch (DE)**
• **ZUBERBÜHLER, Ulrich**
**70563 Stuttgart (DE)**

(74) Vertreter: **Ter Meer Steinmeister & Partner Patentanwälte mbB**
**Nymphenburger Straße 4**
**80335 München (DE)**

(56) Entgegenhaltungen:
EP-A1- 2 360 230     WO-A1-2014/167477
WO-A1-2014/170200     DE-A1-102006 035 893
US-A1- 2011 041 740

EP 3 390 693 B1

## Beschreibung

### Gebiet der Erfindung

Die Erfindung betrifft ein Verfahren zur Erzeugung von Kohlenstoff-basierten Sekundärenergieträgern oder Basischemikalien durch Kopplung einer Oxyfuel-Verbrennung Kohlenstoff-basierter Brennstoffe und einer Hochtemperatur-Festelektrolyt-Elektrolyse (HT-SOEL), wobei für die Oxyfuel-Verbrennung als Oxidationsmittel der bei der HT-SOEL erzeugte Sauerstoff eingesetzt wird und die aus der Oxyfuel-Verbrennung und/oder aus dem Kathodenraum der HT-SOEL gewonnenen Produktgase sowie gegebenenfalls zusätzlich extern zugeführter Wasserstoff in an sich bekannter Weise exotherm zu Kohlenstoff-basierten Sekundärenergieträgern oder Basischemikalien, wie etwa Methanol oder Kohlenwasserstoffen, wie etwa Methan, umgesetzt werden.

### Technischer Hintergrund

Kohlenstoff-basierte (C-basierte) Brennstoffe umfassen beispielsweise Kohle, Erdgas, Öl, kohlenstoffhaltige Abfälle jeglicher Art, wie etwa Kunststoff oder Hausmüll, sonstige Kohlenwasserstoffe und biogene Brennstoffe.

Biogene Brennstoffe sind Brennstoffe biogener, d.h. biologisch-organischer Herkunft und sind Teil der Biomasse. Biomasse ist die einzige erneuerbare Energiequelle, die Kohlenstoff enthält und daher prädestiniert ist für eine nachhaltige Erzeugung von Kohlenstoff-basierten (C-basierten) Sekundärenergieträgern, die vor allem in der Mobilität und chemischen Industrie nicht durch C-freie Energieträger ersetzt werden können.

Das technisch verfügbare Biomassepotential zur Herstellung von C-basierten Sekundärenergieträgern oder Basischemikalien ist limitiert. Ferner ist es aufgrund des geringen Wasserstoffanteils in der Biomasse mit konventionellen Verfahren nicht möglich, 100% des Kohlenstoffs in einen C-basierten Sekundärenergieträger zu überführen. Durch Einkopplung von elektrolytisch erzeugtem Wasserstoff bei der Umwandlung von Biomasse in einen C-basierten Sekundärenergieträger kann ein wesentlich höherer Ertrag an C-basierten Sekundärenergieträgern produziert werden.

### Stand der Technik

Die verfügbaren Verfahren zur Erzeugung von C-basierten Sekundärenergieträgern aus C-basierten Brennstoffen unterscheiden sich im Wesentlichen in der Konversionsrate des in den C-basierten Brennstoffen enthaltenen Kohlenstoffs und somit im Kohlenstoff-Konversionswirkungsgrad.

Die WO 2010/115983 A1 beschreibt ein Energieversorgungssystem mit einer Stromerzeugungseinrichtung zur regenerativen Erzeugung von in ein Stromversorgungsnetz einspeisbarer elektrischer Energie, einer Wasserstofferzeugungseinrichtung unter Verwendung von elektrischer Energie der regenerativen Stromerzeugungseinrichtung, einer Methanisierungseinrichtung, in welcher der in der Wasserstofferzeugungseinrichtung erzeugte Wasserstoff und ein separat zugeführtes Kohlenoxidgas in ein methanhaltiges Gas umgewandelt werden, und einer Gasbereitstellungseinrichtung zur Bereitstellung eines Zusatzgases oder Austauschgases unter Verwendung des methanhaltigen Gases aus der Methanisierungseinrichtung. Ferner wird ein Betriebsverfahren für ein derartiges Energieversorgungssystem beschrieben. Bei diesem sogenannten "Power-to-Gas"-Verfahren ergibt sich ein Gesamtwirkungsgrad von beispielsweise Biogas-$CO_2$ zu Methan (auch als "SNG" = Substitute oder Synthetic Natural Gas bezeichnet) von maximal ca. 60%, da hier für die Wasserstofferzeugung eine Niedertemperatur-Wasserelektrolyse eingesetzt wird.

Die EP 2 360 230 A1 beschreibt ein Verfahren und eine Vorrichtung zur Verwertung von Emissionen eines Kraftwerks, das mit einem Brennstoff auf Kohlenstoffbasis betrieben wird. Das Kraftwerk umfasst mindestens einen Emittenten, welcher Emissionen umfassend ein Kohlenstoffdioxid enthaltendes Abgas erzeugt, mindestens eine Elektrolyseeinrichtung zur Zerlegung von Wasser in Wasserstoff und Sauerstoff, und mindestens einen, dem mindestens einen Emittenten und der mindestens einen Elektrolyseeinrichtung nachgeschalteten chemischen Reaktor zur Umsetzung zumindest eines Teils des Abgases und des Wasserstoffs in mindestens einen synthetischen Grund- oder Brennstoff. Bei diesen Verfahren wird der mindestens einen Elektrolyseeinrichtung und/oder dem mindestens einen chemischen Reaktor zusätzlich zu einer ersten Emission umfassend mindestens den Teil des Abgases, mindestens eine zweite Emission des mindestens einen Emittenten zur Verwertung zugeführt. Die Elektrolyseeinrichtung ist vorzugsweise eine Hochtemperatur-Elektrolyseeinheit mit einer Betriebstemperatur von mindestens 600°C, welche mit Wasser in dampfförmigem Zustand gespeist wird. Bei einer Dampfeinspeisung wird hierbei von einem elektrischen Wirkungsgrad von lediglich bis zu 80% ausgegangen, was einem exothermen Betrieb der Hochtemperatur-Elektrolyse entspricht. Die Temperaturniveaus der verschiedenen Abwärmeströme des in dieser Druckschrift beschriebenen Verfahrens werden nicht erwähnt.

Die DE 10 2006 035 893 A1 beschreibt ein Verfahren zur Wiederaufarbeitung der Verbrennungsprodukte Kohlendioxid und Wasser in erneuerbare synthetische Brenn- und Kraftstoffe mit Hilfe elektrischer Energie, bei dem durch Elektrolyse von Wasser, vorzugsweise Wasserdampf, hergestellter Wasserstoff mit Kohlendioxid zu einem Koh-

# EP 3 390 693 B1

lendioxid-Wasserstoff-Gemisch bis zu einem Molverhältnis von 1 zu 3,5 vermischt, dieses in einem Hochtemperaturrekuperator vorgewärmt und danach in einer elektrisch beheizten Vorrichtung oder einem elektrischen Plasmagenerator auf hohe Temperaturen erhitzt wird. Das sich bildende Syntheserohgas wird rekuperativ zur Vorwärmung des Kohlendioxid-Wasserstoffgemisches genutzt. Da bei diesem Verfahren eine Hochtemperatur-Wasserdampfelektrolyse eingesetzt wird, kann ein Gesamtwirkungsgrad von beispielsweise Biogas-$CO_2$ zu Methan (SNG) von ca. 80% erreicht werden.

WO 2014/167477 A1, WO 2014/170200 A1 und US 2011/0041740 beschreiben weitere Verfahren und Vorrichtungen.

Die im Stand der Technik bekannten Biomasse-Konversionsverfahren sind mit verschiedenen Nachteilen verbunden.

Im Falle der anaeroben Biogaserzeugung erfolgt keine Nutzung des bei der Wasserelektrolyse entstehenden Sauerstoffs. Ferner wird trotz Einkopplung von Elektrolyse-Wasserstoff nur eine unvollständige Kohlenstoffkonversion erreicht, da der kohlenstoffhaltige Gärrest nicht genutzt werden kann.

Im Falle der thermochemischen Vergasung besteht ein hoher Bedarf an erneuerbarer elektrischer Energie, die für die Bereitstellung von Wasserstoff aus der Wasserelektrolyse für eine möglichst hohe Kohlenstoffkonversion in einen Kohlenstoff-basierten Sekundärenergieträger benötigt wird.

Die bei der exothermen Synthese von Kohlenstoff-basierten Sekundärenergieträgern anfallende Wärme weist ein Temperaturniveau von typischerweise etwa 250°C auf. Im Stand der Technik beschränkt sich die bislang erzielte Wärmeintegration aus dieser Abwärme auf die Verdampfungsenthalpie zur Wasserdampferzeugung für die Hochtemperatur-Wasserelektrolyse.

## Aufgabe der Erfindung

Der Erfindung liegt die Aufgabe zugrunde, unter Vermeidung der Nachteile des Standes der Technik ein effizientes und einfaches Verfahren zur Erzeugung von Kohlenstoff-basierten Sekundärenergieträgern oder Basischemikalien durch Kopplung einer Oxyfuel-Verbrennung C-basierter Brennstoffe und einer Hochtemperatur-Festelektrolyt-Elektrolyse vorzusehen, die eine möglichst vollständige Konversion des in den Brennstoffen enthaltenen Kohlenstoffs zu Kohlenstoff-basierten Sekundärenergieträgern oder Basischemikalien ermöglicht. Hierbei soll die Hochtemperatur-Festelektrolyt-Elektrolyse mit einem reduzierten elektrischen Energiebedarf durchführbar sein, um so den Gesamtwirkungsgrad des Kohlenstoff-Konversionsverfahrens zu steigern.

## Zusammenfassung der Erfindung

Die obige Aufgabe wird gelöst durch ein Verfahren zur Erzeugung von C-basierten Sekundärenergieträgern gemäß Anspruch 1. Bevorzugte bzw. besonders zweckmäßige Ausgestaltungen der Erfindung sind in den Unteransprüchen angegeben.

## Detaillierte Beschreibung der Erfindung

Das erfindungsgemäße Verfahren ermöglicht eine gesteigerte bis vollständige Konversion des in den C-basierten Brennstoffen enthaltenen Kohlenstoffs - über die Stufe als Kohlendioxid - in Kohlenstoff-basierte Sekundärenergieträger oder Basischemikalien. Hierbei kann der gesamte Sauerstoffbedarf für die Oxyfuel-Verbrennung aus der Hochtemperatur-Festelektrolyt-Elektrolyse bereitgestellt werden.

Unter Oxyfuel-Verbrennung wird hierin die Verbrennung eines C-basierten Brennstoffs in einem Gasgemisch von Sauerstoff und vorzugsweise recycliertem Rauchgas (Recycle Loop), bestehend somit im Wesentlichen aus Sauerstoff, Wasserdampf und Kohlendioxid, verstanden. Der Sauerstoffbedarf bzw. das Stöchiometrieverhältnis der Oxyfuel-Verbrennung wird als $\lambda$ definiert.

$$\lambda = \text{Massenstrom Sauerstoff-Zugabe/Massenstrom Sauerstoff, stöchiometrisch}$$

Daraus ergibt sich, dass für $\lambda = 1$ die für die Verbrennung notwendige, stöchiometrische Sauerstoffmenge zugegeben wird.

Das erfindungsgemäße Verfahren wird geeigneterweise im Bereich von $0,8 < \lambda < 1,2$, vorzugsweise $0,95 < \lambda < 1,05$, eingestellt.

Die Oxyfuel-Verbrennung ist gegenüber der Vergasung eine technisch deutlich einfacher zu beherrschende Technologie. Die erfindungsgemäß erzielbaren, deutlich höheren Gesamtwirkungsgrade von den C-basierten Brennstoffen und Strom zum Kohlenstoff-basierten Sekundärenergieträger werden überraschenderweise dadurch erzielt, dass

3

beim erfindungsgemäßen Verfahren Wärme auf unterschiedlichen Temperaturniveaus ausgekoppelt wird, insbesondere dadurch, dass ein Teil der für die Hochtemperatur-FestelektrolytElektrolyse erforderlichen elektrischen Energie durch Hochtemperaturwärme substituiert wird, beim erfindungsgemäßen Verfahren auch als "Abwärme III" bezeichnet, aus der Oxyfuel-Verbrennung der C-basierten Brennstoffe. Hierzu ist es erforderlich, die Hochtemperatur-Festelektrolyt-Elektrolyse im endothermen Betrieb zu fahren, in dem der Betriebspunkt für die pro Elektrolysezelle angelegte Zellspannung zwischen dem elektrischen Energiebedarf deltaG und dem gesamten Energiebedarf deltaH liegt. Dadurch resultiert ein hoher elektrischer Wirkungsgrad ($\eta_{Elektr}$) der Hochtemperatur-Festelektrolyt-Elektrolyse von über 100% bis 140 %, definiert als

$$\eta_{Elektr} = (\ V'_{H2}\ [m^3{}_{STP}\ /\ h]\ *\ LHV_{H2}\ [kWh\ /\ m^3{}_{STP}]\ )\ /\ P_{el}\ [kW]\ *\ 100\ [\%],$$

wobei $V'_{H2}$ den $H_2$-Volumenstrom, $LHV_{H2}$ den unteren Heizwert (Lower Heating Value) des erzeugten Wasserstoffs, STP Standard Temperature and Pressure, and $P_{e1}$ die elektrische Leistung bedeuten.

Die Vorteile des erfindungsgemäßen Verfahrens sind somit insbesondere eine nahezu vollständige Konversion des in den Brennstoffen enthaltenen Kohlenstoffs in Kohlenstoff-basierte Energieträger oder Basischemikalien sowie eine deutliche Erhöhung des Gesamtwirkungsgrades des Systems, insbesondere bewirkt durch eine Substitution elektrischer Energie für die Hochtemperatur-Festelektrolyt-Elektrolyse durch Hochtemperatur-Abwärme aus der Oxyfuel-Verbrennung.

Das erfindungsgemäße Verfahren umfasst mehrere Ausführungsformen.

Gemäß einer ersten Ausführungsform werden die Oxyfuel-Verbrennung und die HT-SOEL räumlich getrennt voneinander durchgeführt, wobei die Abwärme III aus der Oxyfuel-Verbrennung indirekt über mindestens einen Wärmeübertrager der HT-SOEL zugeleitet und ein aus der Oxyfuel-Verbrennung gewonnener Kohlendioxid-Massenstrom mit aus der HT-SOEL gewonnenem Wasserstoff zu C-basierten Sekundärenergieträgern oder Basischemikalien umgesetzt wird. Für die indirekte Wärmeübertragung kommen hierbei verschiedene Optionen in Frage. Beispielsweise erfolgt die Einkopplung über Wärmeübertrager vom Oxyfuel-Rauchgasstrom (heiße Seite) in die HT-SOEL (kalte Seite). Ebenfalls denkbar ist der Wärmetransfer über Heat-Pipes bzw. Wärmerohre von dem Oxyfuel-Rauchgasstrom und/oder vom Oxyfuel-Feuerungsraum (heiße Seite) in die HT-SOEL (kalte Seite). Desweiteren denkbar ist ein Wärmetransfer über einen Wärmeübertragerkreislauf von Oxyfuel-Rauchgasstrom und/oder Oxyfuel-Feuerungsraum (heiße Seite) in die HT-SOEL (kalte Seite). Stromabwärts des Wärmeübertragers kann vom Oxyfuel-Rauchgasstrom Wasser abgetrennt werden, um einen reinen Kohlendioxid-Massenstrom zu erzeugen, welcher der exothermen Synthese von C-basierten Sekundärenergieträgern oder Basischemikalien zugeführt wird. Wasserdampf wird der endothermen HT-SOEL zugeführt. Der durch Elektrolyse entstandene Wasserstoff dient für die exotherme Synthese der C-basierten Sekundärenergieträger oder Basischemikalien.

Gemäß einer zweiten Ausführungsform werden die Oxyfuel-Vertrennung und die HT-SOEL räumlich getrennt voneinander durchgeführt, wobei ein aus der Oxyfuel-Verbrennung gewonnenes, Wasserdampf und Kohlendioxid enthaltendes Rauchgas als Kathodeneduktgas und somit die aus der Oxyfuel-Verbrennung gewonnene Abwärme III direkt der HT-SOEL zugeführt, und ein aus der HT-SOEL gewonnene Synsthesegas, umfassend Wasserstoff, Kohlenmonoxid, Kohlendioxid und gegebenenfalls Wasserdampf, zu C-basierten Sekundärenergieträgern oder Basischemikalien umgesetzt wird. Der direkte Wärmetransfer von der Oxyfuel-Verbrennung zu der endothermen HT-Elektrolyse erfolgt somit durch die fühlbare Wärme des Oxyfuel-Rauchgases. Das Oxyfuel-Rauchgas dient hier als Kathodeneduktgas. In der HT-SOEL erfolgt somit neben der Elektrolyse des Wasserdampfes auch eine Elektrolyse von Kohlendioxid. Im Kathodenraum der HT-SOEL laufen dabei die folgenden Reaktionen ab:

    a) $H_2O \rightarrow H_2 + 1/2 O_2$ ($O_2$ wird über den Anodenraum abgeführt)
    b) $CO_2 \rightarrow CO + 1/2 O_2$ ($O_2$ wird über den Anodenraum abgeführt)
    c) $CO_2 + H_2 \rightarrow CO + H_2O$ (Retro-Shift-Reaktion)

Das aus der HT-SOEL gewonnene Synthesegas umfasst somit im Wesentlichen Wasserstoff, Kohlenmonoxid sowie nicht umgesetzten Wasserdampf und Kohlendioxid. Gegebenenfalls kann eine Teilrückführung des Synthesegases als Zusatzbrennstoff inklusive gegebenenfalls Wasserdampf-Kondensation in die Oxyfuel-Verbrennung erfolgen zur gezielten Einstellung der Temperatur und Menge des Oxyfuel-Rauchgasstromes. Dies kann zu einer optimierten Auslegung der endothermen HT-Elektrolyse mit geringem elektrischen Stromverbrauch dienen.

Gemäß einer dritten Ausführungsform sind die Oxyfuel-Verbrennung und die HT-SOEL räumlich integriert, indem die HT-SOEL innerhalb des Brennraums der Oxyfuel-Verbrennung durchgeführt wird. Bei dieser Ausführungsform wird ein aus der Oxyfuel-Verbrennung gewonnenes, Wasserdampf und Kohlendioxid enthaltendes Rauchgas als Kathodeneduktgas und somit die aus der Oxyfuel-Verbrennung gewonnene Abwärme III direkt der HT-SOEL zugeführt, und ein aus der HT-SOEL gewonnenes Synthesegas, umfassend Wasserstoff, Kohlenmonoxid, Kohlendioxid und gegebenenfalls Wasserdampf, sowie zusätzlich extern zugeführter Wasserstoff werden zu C-basierten Sekundärenergieträgern oder Basischemikalien umgesetzt. Bei dieser Ausführungsform wird somit die Hochtemperatur-Abwärme aus der Oxyfuel-Verbrennung direkt über das Rauchgas sowie zusätzlich über Wärmeleitung und Wärmestrahlung transportiert.

Die Anode in der endothermen HT-Elektrolyse entspricht somit der Oxyfuel-Verbrennung. In Kathodenraum der endothermen HT-Elektrolyse wird unterstöchiometrisches Synthesegas erzeugt, das heißt dieses Synthesegas enthält für die nachfolgende Vollkonversion in der exothermen Synthese zu wenig Wasserstoff. Daher ist es erforderlich, zusätzlich Wasserstoff, beispielsweise aus einer weiteren Niedertemperatur (NT) - oder HT-Elektrolyse der exothermen Synthese zuzuführen. Im einfachsten Fall ist die Kathode der HT-SOEL als Rohrbündel ausgestaltet, wobei die Innenfläche der Rohre die Kathode und die Außenfläche die Anode bilden. An der Kathodenseite finden daher folgende Reaktionen statt:

i)

$$H_2O+2e^- \rightarrow H_2+O_2^{2-}$$

ii)

$$CO_2+2e^- \rightarrow CO+O^{2-}$$

An der Anodenseite findet folgende Reaktion statt:

iii)

$$O^{2-} \rightarrow 1/2O_2+2e^-$$

Dies bedeutet, dass Sauerstoff direkt in den Bereich der Anode-Oxyfuel-Verbrennung eintritt und die C-basierten Brennstoffe oxidiert. Der "Sauerstoffgenerator" sitzt somit direkt im Brennraum der Oxyfuel-Verbrennung.

Im Falle der externen Wasserstofferzeugung aus einer weiteren NT- oder HT-Elektrolyse, ist es ebenfalls möglich, den bei dieser externen Elektrolyse anfallenden Sauerstoff ebenfalls in den Bereich der Anode-Oxyfuel-Verbrennung einzuleiten.

Die obigen Ausführungsformen des erfindungsgemäßen Verfahrens können ebenfalls miteinander kombiniert werden.

Beim erfindungsgemäßen Verfahren wird die HT-SOEL vorzugsweise bei Temperaturen von 600 - 1100 °C, weiter vorzugsweise von 800 - 1050 °C, durchgeführt.

Die Abwärme I aus der exothermen Synthese von C-basierten Sekundärenergieträgern oder Basischemikalien liegt vorzugsweise bei einem Temperaturniveau von weniger als 500 °C, typischerweise weniger als 300 °C, insbesondere bevorzugt bei etwa 250 °C.

Die Abwärme II, welche den Produkten Wasserstoff und Sauerstoff aus der HT-SOEL innewohnt, liegt vorzugsweise bei einem Temperaturniveau unterhalb der Betriebstemperatur der HT-SOEL, typischerweise bei Temperaturen von weniger als 800 °C.

Die Hochtemperatur-Abwärme aus der Oxyfuel-Verbrennung, das heißt die Abwärme III, liegt vorzugsweise bei einem Temperaturniveau von oberhalb der Betriebstemperatur der HT-SOEL, weiter vorzugsweise von über 800 °C. Das Abgas aus der Oxyfuel-Verbrennung ist im Wesentlichen ein $CO_2/H_2O$-Rauchgas, aus dem sich durch Auskondensieren von $H_2O$ auf einfache Weise ein reiner $CO_2$-Massenstrom erzeugen lässt. Dieses $CO_2$ wird gemäß der ersten Ausführungsform des erfindungsgemäßen Verfahrens mit dem bei der HT-SOEL anfallenden $H_2$ zu C-basierten Kraftstoffen oder Grundchemikalien umgesetzt. Hierbei kann der Kohlenstoff aus dem C-basierten Brennstoff zu nahezu 100 % in einen C-basierten Kraftstoff konvertiert werden. Gegenüber der konventionellen Erzeugung von Biokraftstoffen wird hierbei der Kraftstoffertrag um ein Mehrfaches gesteigert. Bei herkömmlichem Bio-Diesel oder Bioethanol wird nur ca. 1/4 des Biomasse-Kohlenstoffs zu Kraftstoff-Kohlenstoff konvertiert.

Beim erfindungsgemäßen Verfahren ist es möglich, den gesamten Sauerstoffbedarf für die Oxyfuel-Verbrennung durch die HT-SOEL zu decken. Dies hat den Vorteil, einen $H_2O/CO_2$-Strom für die HT-SOEL und einen $H_2O/H_2/CO_2/CO$-Strom für die Synthese zu generieren.

Beim erfindungsgemäßen Verfahren können alle üblichen C-basierten Brennstoffe eingesetzt werden. Vorzugsweise werden biogene Brennstoffe eingesetzt.

Als biogene Brennstoffe können Festbrennstoffe, wie Brennholz, Gärrest oder Klärschlamm und/oder Flüssigbrennstoffe, wie etwa Pflanzenöle einschließlich Slurries, wie etwa Biopyrolyseöl/Koks und/oder Brenngase, wie Faulgase oder Klärgase, eingesetzt werden.

Die elektrische Energie für die HT-SOEL stammt vorzugsweise aus einer regenerativen Stromerzeugung. Für die regenerative Stromerzeugung können übliche Anlagen eingesetzt werden, wie Windkraftanlagen, Photovoltaikanlagen, Geothermiekraftwerke, Biomassekraftwerke, Wasserkraftwerke, Solarthermiekraftwerke oder Kombinationen daraus.

Als Kohlenstoff-basierte Sekundärenergieträger oder Basischemikalien werden insbesondere Kohlenwasserstoffe, wie etwa Methan oder Fischer-Tropsch-Kohlenwasserstoffe oder chemische Verbindungen, bestehend aus Kohlenstoff, Wasserstoff und Sauerstoff, wie beispielsweise Methanol, Ethanol oder Dimethylether, hergestellt. Die Synthese der C-basierten Sekundärenergieträger oder Basischemikalien erfolgt nach üblichen, dem Fachmann bekannten Methoden, beispielsweise bei Temperaturen im Bereich von 200 °C-600 °C.

## Kurze Beschreibung der Figuren

**Fig.** 1 zeigt die schematische Darstellung einer ersten Ausführungsform des erfindungsgemäßen Verfahrens.

Fig. 2 zeigt die schematische Darstellung einer zweiten Ausführungsform des erfindungsgemäßen Verfahrens.

Fig. 3 zeigt die schematische Darstellung einer dritten Ausführungsform des erfindungsgemäßen Verfahrens.

Fig. 4 zeigt ein Diagramm der thermodynamischen Parameter für die Elektrolyse von Wasser/Wasserdampf.

Fig. 5 zeigt ein Diagramm der thermodynamischen Parameter für die Elektrolyse von Kohlendioxid.

## Beschreibung der bevorzugten Ausführungsformen

Bevorzugte Ausführungsformen des erfindungsgemäßen Verfahrens werden unter Bezugnahme auf die beigefügten Figuren nachfolgend näher erläutert.

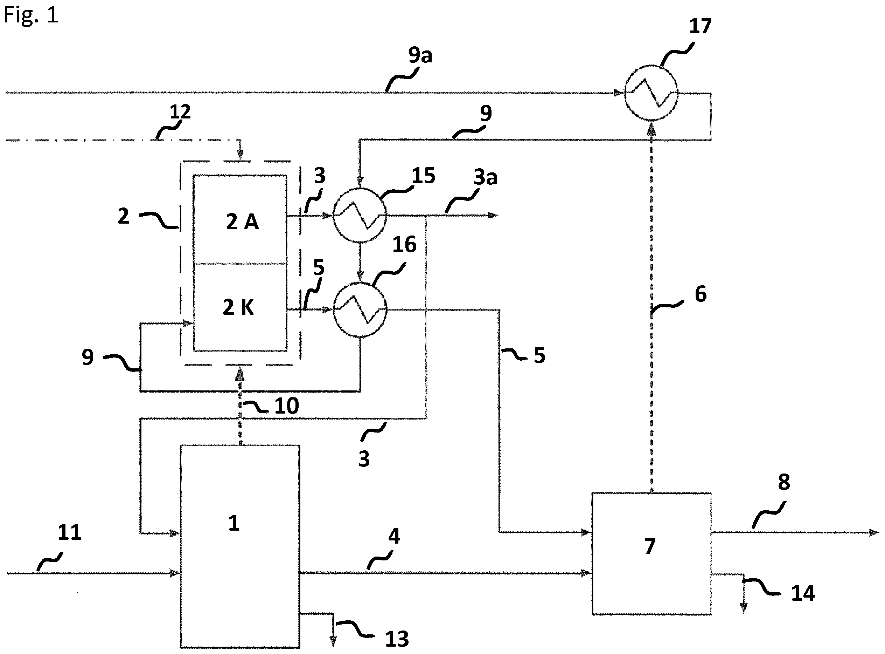

**Fig. 1** zeigt die schematische Darstellung einer ersten Ausführungsform des erfindungsgemäßen Verfahrens. Hierbei ist eine Oxyfuel-Verbrennungseinrichtung 1 mit einer Hochtemperatur-Festelektrolyt-Elektrolyseeinrichtung 2 mit Anodenraum 2A und Kathodenraum 2K (HT-SOEL) gekoppelt. Der Oxyfuel-Verbrennungseinrichtung 1 werden über 11 C-basierte Brennstoffe zugeführt. Die Oxyfuel-Verbrennungseinrichtung 1 beinhaltet typischerweise Komponenten, wie einen Recycle-Loop (nicht gezeigt) zur Temperierung der Oxyfuel-Verbrennung und Einstellung des Verbrennungsedukts (Verhältnis von Elektrolyse-Sauerstoff 3 aus der HT-SOEL, Kohlendioxid und Wasserdampf), eine Vorrichtung zur Abtrennung von Asche und sonstigen Feststoffpartikeln über 13 sowie gegebenenfalls eine Vorrichtung für die Dampf-Kondensation (nicht gezeigt) aus dem Oxyfuel-Rauchgas 4. Die HT-SOEL 2 zur Elektrolyse von Wasserdampf 9 wird endotherm betrieben. Die HT-SOEL 2 beinhaltet die Erhitzung von Wasserdampf 9 durch Wärmerekuperation über Wärmeübertrager 15, 16 von Elektrolyse-Wasserstoff 5 und Elektrolyse-Sauerstoff 3. Die Erzeugung von Elektrolyse-Wasserstoff 5 erfolgt in der Kathode 2K und die Erzeugung von Elektrolyse-Sauerstoff 3 erfolgt in der Anode 2A. Der Elektrolyse-Sauerstoff 3 dient als Oxidationsmittel für die Oxyfuel-Verbrennung 1. Überschüssiger Elektrolyse-Sauerstoff kann über 3a abgezogen werden.

Das aus der Oxyfuel-Verbrennung gewonnene Rauchgas bzw. Kohlendioxid 4 wird als Eduktgas der Syntheseeinrichtung 7 zugeführt. In der Syntheseeinrichtung 7 wird das Kohlendioxid 4 und der Elektrolyse-Wasserstoff 5 exotherm zu C-basierten Sekundärenergieträgern oder Basischemikalien 8 umgesetzt. Wasser bzw. Kondensat aus der Synthese 7 kann über 14 abgezogen werden. Über 6 erfolgt ein Wärmetransfer durch Wärmeübertragung beim Wärmeübertrager 17 aus der exothermen Synthese zur Erzeugung von Wasserdampf 9.

Die Syntheseeinrichtung 7 beinhaltet typischerweise Komponenten zur Temperierung und Druckeinstellung des Syntheseedukts, zur Edukterzeugung durch Mischen von Kohlendioxid, Wasserstoff und gegebenenfalls Wasserdampf (zur Vermeidung von Kohlenstoffablagerung), gegebenenfalls zur Wasserdampferzeugung für das Syntheseedukt sowie zur Aufbereitung von Syntheseprodukten (beispielsweise Temperierung und gegebenenfalls Kondensation von Wasserdampf). Bei dem C-basierten Syntheseprodukt kann es sich um Sekundärenergieträger oder um Grundchemikalien, wie beispielsweise Methan, Methanol, Dimethylether, Fischer-Tropsch-Kraftstoffe etc., handeln.

Über 9 erfolgt die Wasserdampfzugabe zur endothermen HT-Elektrolyse inklusive Erhitzung von Wasserdampf durch Wärmerekuperation von Elektrolyse-Sauerstoff über Wärmeübertrager 15 und Elektrolyse-Wasserstoff 5 über Wärmeübertrager 16. Es ist darauf hinzuweisen, dass die Anzahl und Reihenfolge der Wärmeübertrager 15 und 16 beliebig ist. Ebenfalls ist eine Aufsplittung dieser Wärmeübertrager in mehr als 2 Wärmeübertrager möglich. Ebenfalls ist eine Aufsplittung von Wärmeübertrager 17 in mehr als einen Wärmeübertrager möglich.

Der indirekte Wärmetransfer durch Wärmeübertragung 10 aus der Oxyfuel-Verbrennung 1 zu der endothermen HT-Elektrolyse 2 ist über verschiedene Optionen verwirklichbar. Beispielsweise erfolgt die Einkopplung über Wärmeübertrager (nicht gezeigt) vom Oxyfuel-Rauchgasstrom (heiße Seite) in die HT-SOEL (kalte Seite). Ebenfalls möglich ist der Wärmetransfer über Wärmerohre vom Oxyfuel-Rauchgasstrom und/oder vom Oxyfuel-Feuerungsraum (heiße Seite) in die HT-SOEL (kalte Seite). Schließlich kann der Wärmetransfer auch über einen Wärmeübertragerkreislauf von Oxyfuel-Rauchgasstrom und/oder Oxyfuel-Feuerungsraum (heiße Seite) in die HT-SOEL (kalte Seite) erfolgen.

Die Zuleitung von elektrischem Strom zu der HT-SOEL erfolgt über Leitung 12. Hierzu dient vorzugsweise regenerativ erzeugter Strom, beispielsweise aus Windkraft- oder Photovoltaikanlagen.

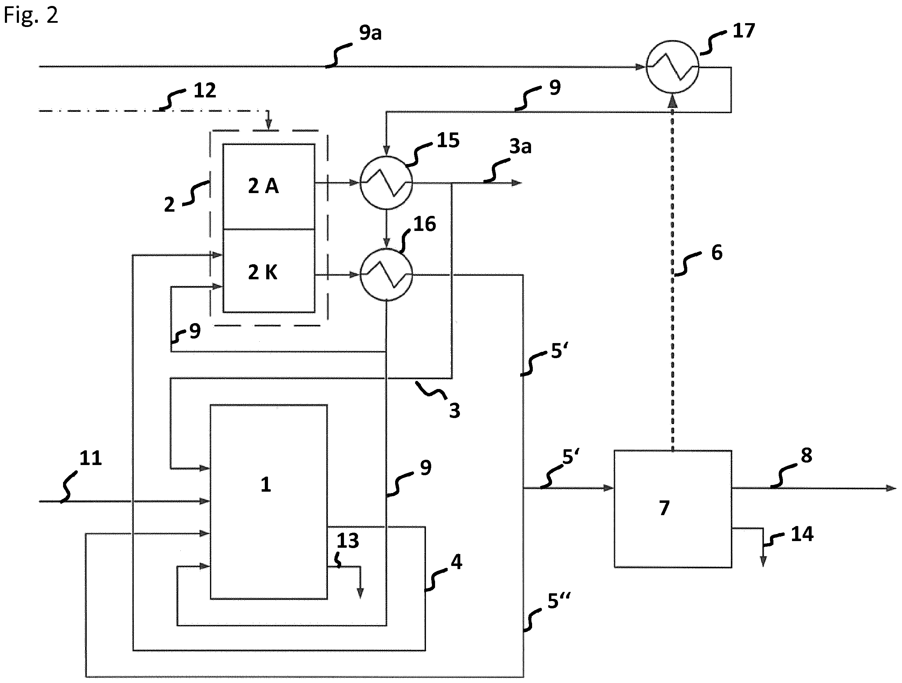

**Fig. 2** zeigt die schematische Darstellung einer zweiten Ausführungsform des erfindungsgemäßen Verfahrens. Hierbei ist eine Oxyfuel-Verbrennungseinrichtung 1 mit einer Hochtemperatur-Festelektrolyt-Elektrolyse 2 mit einer Anodenkammer 2A und einer Kathodenkammer 2K (HT-SOEL) gekoppelt. Der Oxyfuel-Verbrennungseinrichtung 1 werden über 11 C-basierte Brennstoffe zugeführt. Die Oxyfuel-Verbrennungseinrichtung 1 beinhaltet typischerweise Komponenten, wie einen Recycle-Loop (nicht gezeigt) zur Temperierung der Oxyfuel-Verbrennung und Einstellung des Verbrennungsedukts (Verhältnis von Elektrolyse-Sauerstoff, Kohlendioxid und Wasserdampf), eine Einrichtung zur Abtrennung von Asche und sonstigen Partikeln über 13, sowie gegebenenfalls Mittel zur Teilrückführung von Synthesegas 5" als Zusatzbrennstoff zur Einstellung der Temperatur und Menge des Oxyfuel-Rauchgasstroms 4'. Eine Einrichtung zur Kondensation von Wasserdampf aus dem Oxyfuel-Rauchgas 4' ist hier nicht vorgesehen.

Gemäß dieser Ausführungsform wird ein aus der Oxyfuel-Verbrennung 1 gewonnenes, Wasserdampf und Kohlendioxid enthaltendes Rauchgas 4' als Kathodeneduktgas und somit die aus der Oxyfuel-Verbrennung gewonnene Hochtemperatur-Abwärme direkt der Kathodenkammer 2K der HT-SOEL zur endothermen HT-Elektrolyse von Oxyfuel-Rauchgas zugeführt. Ein aus der Kathodenkammer 2K erhaltenes Synthesegas 5', bestehend im Wesentlichen aus Wasserstoff, Kohlenmonoxid sowie nicht umgesetztem Kohlendioxid und gegebenenfalls Wasserdampf, wird der Syntheseeinrichtung 7 für die exotherme Synthese von C-basierten Sekundärenergieträgern oder Basischemikalien zugeführt. Die Syntheseeinrichtung 7 umfasst die gleichen Komponenten und Vorrichtungen wie im Falle der Syntheseeinrichtung 7 der ersten Ausführungsform gemäß Fig. 1.

Der Wärmetransfer durch Wärmeübertragung über 6 und den Wärmeübertrager 17 aus der exothermen Synthese 7 zur Erzeugung von Wasserdampf 9 für die endotherme HT-Elektrolyse 2 erfolgt analog Fig. 1.

Die Vorerhitzung des Wasserdampfes 9 sowohl für die HT-Elektrolyse als auch die Oxyfuel-Verbrennung erfolgt weiterhin durch Wärmerekuperation von Elektrolyse-Sauerstoff 3 über Wärmeübertrager 15 als auch von Elektrolyse-Synthesegas 5' über Wärmeübertrager 16. Die Reihenfolge der Wärmeübertrager 15 und 16 kann beliebig sein. Ebenfalls ist die Anzahl der Wärmeübertrager nicht auf 2 beschränkt. Ferner ist eine Aufsplittung der Wärmeübertrager 15, 16, 17 in mehr als 3 Wärmeübertrager möglich. Wie bei der Ausführungsform in Fig. 1 kann flüssiges Waser über 9a dem Wärmeübertrager 17 zur Erzeugung von Wasserdampf 9 zugeführt werden.

Bei der in Fig. 2 gezeigten Ausführungsform erfolgt gegebenenfalls eine Teilrückführung des Synthesegases über 5" als Zusatzbrennstoff inklusive gegebenenfalls Wasserdampf-Kondensation in die Oxyfuel-Verbrennung 1 zur gezielten Einstellung der Temperatur und Menge des Oxyfuel-Rauchgasstroms 4' für eine optimierte Auslegung der endothermen HT-Elektrolyse mit geringem Stromverbrauch.

Ebenfalls wird wie bei Fig. 1 elektrischer Strom, vorzugsweise regenerativ erzeugter Strom, über Leitung 12 der HT-SOEL zugeführt.

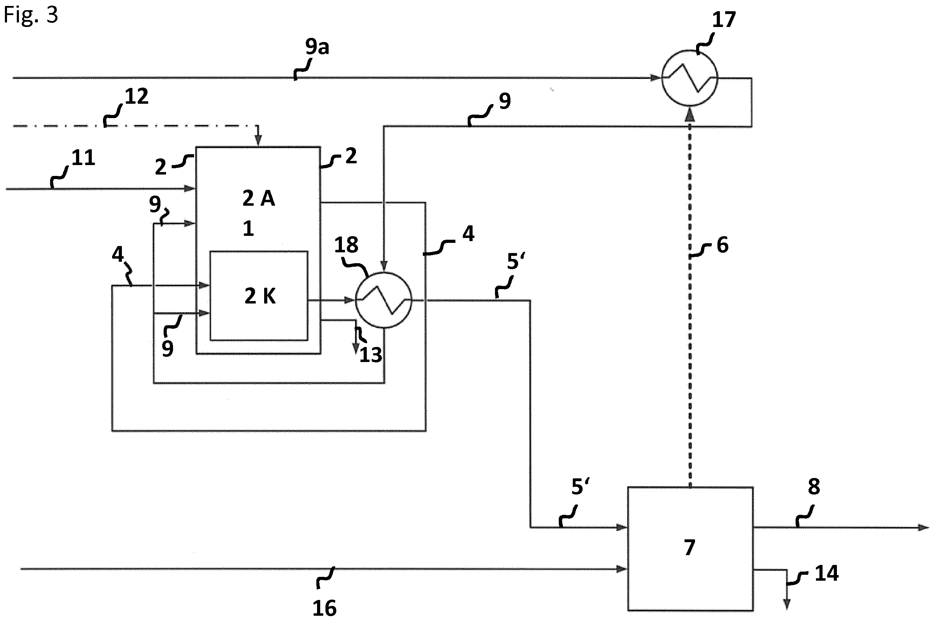

**Fig. 3** zeigt eine schematische Darstellung einer dritten Ausführungsform des erfindungsgemäßen Verfahrens. Bei dieser Ausführungsform sind die Oxyfuel-Verbrennung 1 und die HT-SOEL 2 räumlich integriert, in dem die HT-SOEL innerhalb des Brennraums der Oxyfuel-Verbrennungseinrichtung 1 durchgeführt wird. Hierbei entspricht also die Anode 2A der endothermen HT-Elektrolyse der Oxyfuel-Verbrennung 1. Die Anode 2A der HT-SOEL mit integrierter Oxyfuel-Verbrennung beinhaltet typischerweise Komponenten, wie einen Recycle-Loop (nicht gezeigt) zur Temperierung der Oxyfuel-Verbrennung und Einstellung des Verbrennungsedukts (Verhältnis von Elektrolyse-Sauerstoff, Kohlendioxid und Wasserdampf), eine Vorrichtung zur Abtrennung von Asche und sonstigen Partikeln über 13. Es erfolgt ein direkter Wärmetransfer von der Anodenseite 2A der Oxyfuel-Verbrennung zum Kathodenraum 2K durch die fühlbare Wärme des Kohlendioxid und Wasserdampf enthaltenden, vorzugsweise partikelgereinigten Oxyfuel-Rauchgases 4' sowie durch Wärmestrahlung und Wärmeleitung zur Kathodenseite 2K. Im Kathodenraum 2K der HT-SOEL erfolgt eine Elektrolyse von Oxyfüel-Rauchgas 4' zur Erzeugung eines Synthesegases 5', umfassend im Wesentlichen Wasserstoff, Kohlenmonoxid, Kohlendioxid und gegebenenfalls Wasserdampf. Hierbei wird im Kathodenraum 2K unterstöchiometrisches Synthesegas erzeugt, da dieses zu wenig Wasserstoff für eine Vollkonversion in der SyntheseEinrichtung 7 enthält. Daher ist es erforderlich, zusätzlich Wasserstoff 16 aus einer weiteren NT- oder HT-Elektrolyse als Zusatz-Edukt der Synthese zur Bereitstellung eines stöchiometrischen Syntheseedukts einzubringen.

Infolge einer stöchiometrischen Oxyfuel-Verbrennung von C-basiertem Brennstoff 11 wird auf der Anodenseite 2A kein Sauerstoffüberschuss erzielt. Neben Wasserdampf wird somit das im Oxyfüel-Rauchgas 4' enthaltene Kohlendioxid als Eduktgas der Kathode umgesetzt zur Erzielung eines Synthesegases aus Wasserstoff, Kohlenmonoxid und nicht umgesetztem Wasserdampf und Kohlendioxid.

Die exotherme Synthese von C-basierten Sekudärenergieträgern oder Basischemikalien 8 in der Syntheseeinrichtung 7 erfolgt analog der in Fig. 2 beschriebenen Ausführungsform. Ebenfalls erfolgt über 6 ein Wärmetransfer durch Wärmeübertragung beim Wärmeübertrager 17 zur Erzeugung von Wasserdampf 9. Es erfolgt eine Zuführung von Wasserdampf 9 zur Oxyfuel-Verbrennung inklusive einer Vorerhitzung des Wasserdampfes durch Wärmerekuperation über den Wärmeübertrager 18 von Synthesegas 5', das der Kathodenseite als Produktgas entnommen wird. Wasserdampf 9 kann sowohl der Kathode 2K als auch der Anode 2A zugeführt werden zur gezielten Einstellung der Temperatur und für eine optimierte Auslegung der endothermen HT-Elektrolyse mit geringem Stromverbrauch. Der Wasserdampf 9 kann anstatt zur Anode 2A auch nur zur Kathode 2K geleitet werden. In diesem Fall kann ebenfalls mit dem entstehenden Synthesegas 5' am Kathodenaustritt vorerhitzt werden. Auch hier ist die Anzahl und Reihenfolge von Wärmeübertragern beliebig. Ebenfalls ist eine Aufsplittung der Wärmeübertrager 17 und 18 auf mehrere Wärmeübertrager möglich.

Wie bei den Figuren 1 und 2 kann die Zugabe von flüssigem Wasser 9a über den Wärmeübertrager 17 erfolgen. Die Zugabe von C-basierten Brennstoffen 11 sowie die Zuführung von elektrischem Strom 12 erfolgen in einer zu den Figuren 1 und 2 analogen Weise.

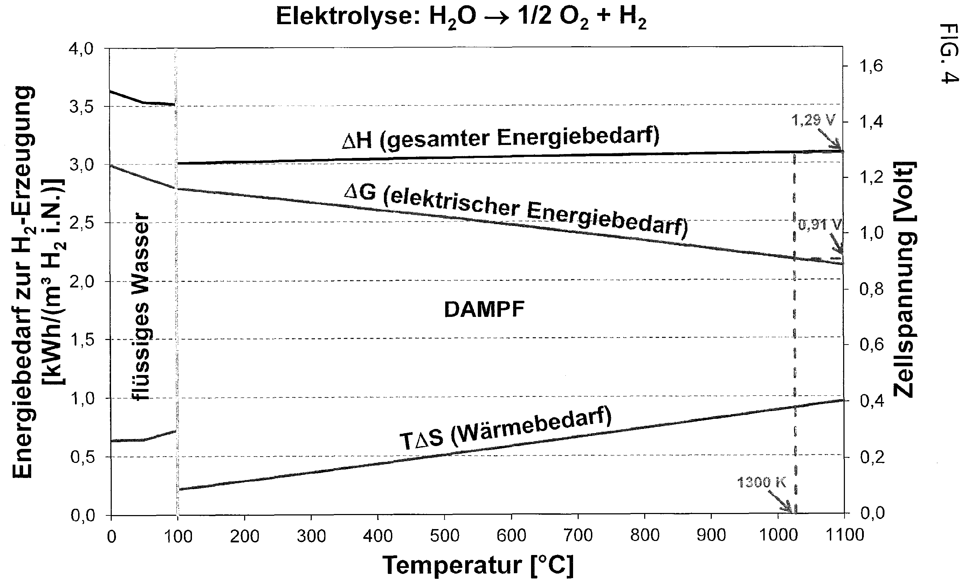

Fig. 4 zeigt ein Diagramm der thermodynamischen Parameter für die Elektrolyse von Wasser/Wasserdampf. In diesem Diagramm sind für das Beispiel einer Temperatur von 1300 K die minimalen Zersetzungsspannungen angegeben. Ein kritischer Punkt des erfindungsgemäßen Verfahrens besteht darin, dass die HT-Elektrolyse im endothermen Betrieb gefahren wird. Dies bedeutet, dass gemäß diesem Diagramm der Betriebspunkt für die pro Elektrolysezelle angelegte Zellspannung zwischen dem elektrischen Energiebedarf deltaG und dem gesamten Energiebedarf deltaH liegt. Für das gezeigte Beispiel von 1300 K liegt die angelegte Zellspannung somit zwischen 0,91 und 1,29 V. Bei einer Zellspannung von > 1,29 V würde der Betrieb exotherm und die HT-Elektrolyse würde somit Wärme produzieren. Somit wäre eine externe Wärme nicht mehr einkoppelbar, um den elektrischen Energiebedarf der HT-Elektrolyse zu senken. Entscheidend beim erfindungsgemäßen Verfahren ist somit, dass der fehlende Energiebedarf der HT-SOEL im endothermen Betrieb durch die Abwärme aus der Oxyfuel-Verbrennung gedeckt wird.

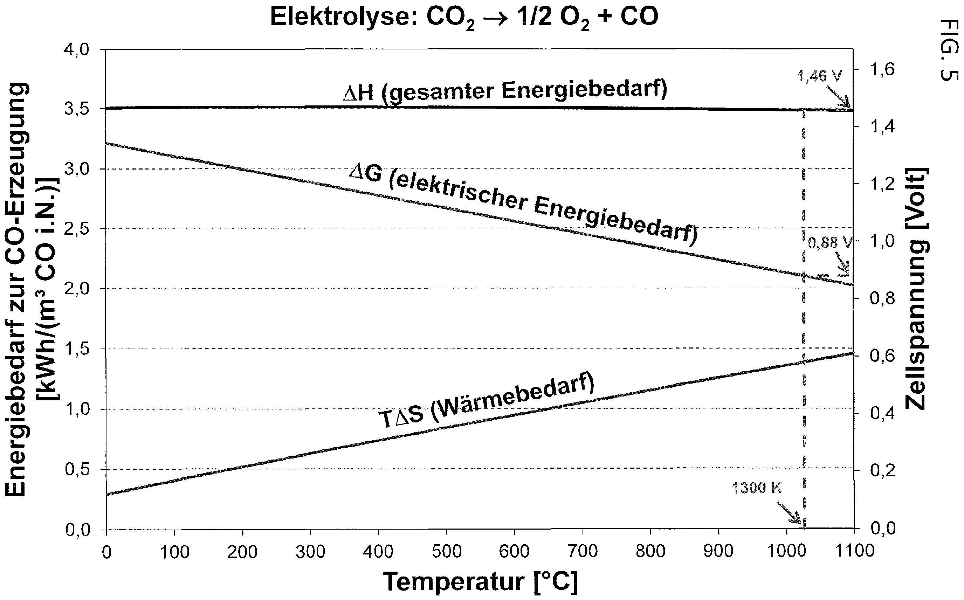

**Fig. 5** zeigt ein Diagramm der thermodynamischen Parameter für die Elektrolyse von Kohlendioxid gemäß der zweiten und dritten Ausführungsform des erfindungsgemäßen Verfahrens. Um die HT-Elektrolyse im endothermen Betrieb fahren zu können, ist es auch hier erforderlich, dass bei dem gezeigten Beispiel von 1300 K der Betriebspunkt für die pro Elektrolysezelle angelegte Zellspannung zwischen dem elektrischen Energiebedarf deltaG und dem gesamten Energiebedarf deltaH liegt. In diesem Fall muss die pro Elektrolysezelle angelegte Zellspannung zwischen 0,88 und 1,46 V liegen, um einen endothermen Betrieb zu gewährleisten. Bei > 1,46 V wird der Betrieb exotherm, sodass externe Wärme nicht mehr einkoppelbar ist.

**Patentansprüche**

1. Verfahren zur Erzeugung von C-basierten Sekundärenergieträgern oder Basischemikalien (8) durch Kopplung einer Oxyfuel-Verbrennung (1) C-basierter Brennstoffe (11) und einer Hochtemperatur-Festelektrolyt-Elektrolyse (HT-SOEL) (2, 2A, 2K), wobei für die Oxyfuel-Verbrennung als Oxidationsmittel aus der HT-SOEL erzeugter Sauerstoff (3) eingesetzt wird und die aus der Oxyfuel-Verbrennung und/oder aus dem Kathodenraum (2K) der HT-SOEL gewonnenen Produktgase (4, 4', 5, 5') sowie gegebenenfalls zusätzlich extern zugeführter Wasserstoff (16) in an sich bekannter Weise exotherm zu C-basierten Sekundärenergieträgern oder Basischemikalien (8) umgesetzt werden,

   wobei bei dem Verfahren Wärme auf unterschiedlichen Temperaturniveaus in der Weise ausgekoppelt wird, dass eine Abwärme I (6) aus der exothermen Synthese (7) von C-basierten Sekundärenergieträgern oder Basischemikalien (8) zur Wasserdampferzeugung für die HT-SOEL (2, 2A, 2K) verwendet wird, eine den Produktgasen (3, 4, 4', 5, 5') der HT-SOEL innewohnende Abwärme II zur Vorheizung des der HT-SOEL zuzuführenden Wasserdampfes (9) verwendet wird, und eine aus der Oxyfuel-Verbrennung gewonnene Abwärme III (10) der HT-SOEL (2, 2A, 2K) zugeleitet wird,

   wobei die HT-SOEL im endothermen Betrieb gefahren wird, in dem der Betriebspunkt für die pro Elektrolysezelle angelegte Zellspannung zwischen dem elektrischen Energiebedarf deltaG und dem gesamten Energiebedarf deltaH liegt und der fehlende Energiebedarf der HT-SOEL im endothermen Betrieb durch die Abwärme III (10) gedeckt wird, und wobei der elektrische Wirkungsgrad bei der HT- SOEL mehr als 100 % und bis zu 140 % beträgt, wenn der elektrische Wirkungsgrad ($\eta_{Elektr}$) der HT- SOEL gemäß nachfolgender Formel (I) definiert ist

$$\eta_{Elektr} = (\ V'_{H2}\ [m^3{}_{STP}\ /\ h]\ *\ LHV_{H2}\ [kWh\ /\ m^3{}_{STP}]\ )\ /\ P_{el}\ [kW]\ *\ 100\ [\%] \qquad (I)$$

   wobei $V'_{H2}$ den $H_2$-Volumenstrom, $LHV_{H2}$ den unteren Heizwert (Lower Heating Value) des erzeugten Wasserstoffs, STP Standard Temperature and Pressure, and $P_{e1}$ die elektrische Leistung bedeuten.

2. Verfahren nach Anspruch 1, wobei die Oxyfuel-Verbrennung (1) und die HT-SOEL (2, 2A, 2K) räumlich getrennt voneinander durchgeführt werden, die Abwärme III (10) aus der Oxyfuel-Verbrennung indirekt über mindestens einen Wärmeübertrager der HT-SOEL zugeleitet wird und ein aus der Oxyfuel-Verbrennung gewonnener Kohlendioxid-Massenstrom (4) mit aus der HT-SOEL gewonnenem Wasserstoff (5) zu C-basierten Sekundärenergieträgern oder Basischemikalien (8) umgesetzt wird.

3. Verfahren nach Anspruch 1, wobei die Oxyfuel-Verbrennung (1) und die HT-SOEL (2, 2A, 2K) räumlich getrennt voneinander durchgeführt werden, ein aus der Oxyfuel-Verbrennung gewonnenes, Wasserdampf und Kohlendioxid enthaltendes Rauchgas (4') als Kathodeneduktgas und somit die aus der Oxyfuel-Verbrennung gewonnene Abwärme III direkt der HT-SOEL (2K) zugeführt wird, und ein aus der HT-SOEL gewonnenes Synthesegas (5'), umfassend Wasserstoff, Kohlenmonoxid, Kohlendioxid und gegebenenfalls Wasserdampf, zu C-basierten Sekundärenergieträgern oder Basischemikalien (8) umgesetzt wird.

4. Verfahren nach Anspruch 1, wobei die Oxyfuel-Verbrennung (1) und die HT-SOEL (2, 2A, 2K) räumlich integriert werden, in dem die HT-SOEL innerhalb des Brennraums der Oxyfuel-Verbrennung durchgeführt wird, ein aus der Oxyfuel-Verbrennung gewonnenes, Wasserdampf und Kohlendioxid enthaltendes Rauchgas (4') als Kathodeneduktgas und somit die aus der Oxyfuel-Verbrennung gewonnene Abwärme III direkt der HT-SOEL (2K) zugeführt wird, und ein aus der HT-SOEL gewonnenes Synthesegas (5'), umfassend Wasserstoff, Kohlenmonoxid, Kohlendioxid und gegebenenfalls Wasserdampf, sowie zusätzlich extern zugeführter Wasserstoff (16) zu C-basierten Sekundärenergieträgern oder Basischemikalien (8) umgesetzt werden.

5. Verfahren nach mindestens einem der vorangehenden Ansprüche , wobei die HT-SOEL (2) bei Temperaturen von 600 - 1100 °C, vorzugsweise von 800 - 1050 °C, betrieben wird.

6. Verfahren nach mindestens einem der vorangehenden Ansprüche, wobei die Abwärme I (6) ein Temperaturniveau von < 500 °C, vorzugsweise etwa 250 °C, aufweist.

7. Verfahren nach mindestens einem der vorangehenden Ansprüche, wobei die Abwärme II ein Temperaturniveau von unterhalb der Betriebstemperatur der HT-SOEL aufweist.

8. Verfahren nach mindestens einem der vorangehenden Ansprüche, wobei die Abwärme III (10) ein Temperaturniveau von oberhalb der Betriebstemperatur der HT-SOEL, typischerweise von über 800 °C, vorzugsweise von über 800 - 1000 °C, aufweist.

9. Verfahren nach mindestens einem der vorangehenden Ansprüche, wobei der gesamte Sauerstoffbedarf für die Oxyfuel-Verbrennung (1) durch die HT-SOEL (2) gedeckt wird.

10. Verfahren nach mindestens einem der vorangehenden Ansprüche, wobei als C-basierte Brennstoffe (11) biogene Brennstoffe (11) in Form von Festbrennstoffen und/oder Flüssigbrennstoffen und/oder Brenngasen eingesetzt werden, wobei die Festbrennstoffe Brennholz, Gärrest oder Klärschlamm sind, wobei die Flüssigbrennstoffe Pflanzenöle sind, und wobei die Brenngase Faulgase oder Klärgase sind.

11. Verfahren nach mindestens einem der vorangehenden Ansprüche, wobei als elektrische Energie (12) für die HT-SOEL (2) solche aus einer regenerativen Stromerzeugung eingesetzt wird.

12. Verfahren nach Anspruch 11, wobei die regenerative Stromerzeugung mittels Windkraftanlagen, Photovoltaikanlagen, Geothermiekraftwerken, Biomassekraftwerken, Wasserkraftwerken, Solarthermiekraftwerken oder Kombinationen daraus erfolgt.

13. Verfahren nach mindestens einem der vorgehenden Ansprüche, wobei als C-basierte Sekundärenergieträger oder Basischemikalien Kohlenwasserstoffe und/oder chemische Verbindungen, bestehend aus Kohlenstoff, Wasserstoff und Sauerstoff erzeugt werden.

**Claims**

1. Process for generating carbon-based secondary energy carriers or base chemicals (8) by coupling oxyfuel combustion (1) of carbon-based fuels (11) and high-temperature solid electrolyte electrolysis (HT-SOEL) (2, 2A, 2K), wherein, for the oxyfuel combustion, oxygen generated from the HT-SOEL is used as an oxidizing agent (3), and the product gases (4, 4', 5, 5) obtained from the oxyfuel combustion and/or from the cathode chamber (2K) of the HT-SOEL, and, optionally, additionally externally supplied hydrogen (16), are reacted exothermically in a manner known per se to form carbon-based secondary energy carriers or base chemicals (8),
wherein, in the process, heat is coupled out at different temperature levels in such a way that waste heat I (6) from the exothermic synthesis (7) of carbon-based secondary energy carriers or base chemicals (8) is used to generate steam for the HT-SOEL (2, 2A, 2K), waste heat II inherent in the product gases (3, 4, 4', 5, 5') of the HT- SOEL is used to preheat the steam (9) to be supplied to the HT-SOEL, and waste heat III (10) obtained from the oxyfuel combustion is fed to the HT-SOEL (2, 2A, 2K),
wherein the HT- SOEL is operated in endothermic operation, in which the operating point for the cell voltage applied per electrolysis cell is between the electrical energy requirement deltaG and the total energy requirement deltaH, and the missing energy requirement of the HT-SOEL in endothermic operation is covered by the waste heat III (10), and wherein the electrical efficiency for the HT-SOEL is more than 100% and up to 140% if the electrical efficiency ($\eta E_{lektr}$) of the HT-SOEL is defined according to the following formula (I)

$$\eta_{Elektr} = (V'_{H2} \ [m^3{}_{STP} / h] * LHV_{H2} \ [kWh / m^3{}_{STP}]) / P_{el} \ [kW] * 100 \ [\%] \qquad (I)$$

wherein $V'_{H2}$ denotes the $H_2$ volume flow, $LHV_{H2}$ denotes the lower heating value of the hydrogen generated, STP denotes the standard temperature and pressure, and $P_{e1}$ denotes the electrical power.

2. Process according to claim 1, wherein the oxyfuel combustion (1) and the HT-SOEL (2, 2A, 2K) are carried out spatially separately from each other, the waste heat III (10) from the oxyfuel combustion is fed indirectly to the HT SOEL via at least one heat exchanger, and a carbon dioxide mass flow (4) obtained from the oxyfuel combustion is reacted with hydrogen (5) obtained from the HT-SOEL to form carbon-based secondary energy carriers or base chemicals (8).

3. Process according to claim 1, wherein the oxyfuel combustion (1) and the HT-SOEL (2, 2A, 2K) are carried out spatially separately from one another, as a cathode reactant gas a flue gas (4') obtained from the oxyfuel combustion and containing steam and carbon dioxide, and thus the waste heat III obtained from the oxyfuel combustion, is supplied directly to the HT-SOEL (2K), and a synthesis gas (5') obtained from the HT-SOEL and comprising hydrogen, carbon monoxide, carbon dioxide and optionally steam is reacted to form carbon-based secondary energy carriers or base chemicals (8).

4. Process according to claim 1, wherein the oxyfuel combustion (1) and the HT-SOEL (2, 2A, 2K) are spatially integrated by the HT-SOEL being carried out within the combustion chamber of the oxyfuel combustion, as the cathode reactant gas a flue gas (4') obtained from the oxyfuel combustion and containing steam and carbon dioxide, and thus the waste heat III obtained from the oxyfuel combustion is supplied directly to the HT-SOEL (2K), and a synthesis gas (5') obtained from the HT-SOEL and comprising hydrogen, carbon monoxide, carbon dioxide and optionally steam, as well as additionally externally supplied hydrogen (16), are reacted to form carbon-based secondary energy carriers or base chemicals (8).

5. Process according to at least one of the preceding claims, wherein the HT-SOEL (2) is operated at temperatures of 600-1100°C, preferably 800-1050°C.

6. Process according to at least one of the preceding claims, wherein the waste heat I (6) has a temperature level of < 500°C, preferably approximately 250°C.

7. Process according to at least one of the preceding claims, wherein the waste heat II has a temperature level below the operating temperature of the HT-SOEL.

8. Process according to at least one of the preceding claims, wherein the waste heat III (10) has a temperature level above the operating temperature of the HT-SOEL, typically above 800°C, preferably above 800-1000°C.

9. Process according to at least one of the preceding claims, wherein the entire oxygen requirement for the oxyfuel combustion (1) is covered by the HT-SOEL (2).

10. Process according to at least one of the preceding claims, wherein biogenic fuels (11) in the form of solid fuels and/or liquid fuels and/or fuel gases are used as the carbon-based fuels (11), wherein the solid fuels are firewood, digestate or sewage sludge, wherein the liquid fuels are vegetable oils, and wherein the fuel gases are digester gases or sewage gases.

11. Process according to at least one of the preceding claims, wherein electrical energy from regenerative power generation is used as electrical energy (12) for the HT-SOEL (2).

12. Process according to claim 11, wherein the regenerative power generation takes place by means of wind power plants, photovoltaic plants, geothermal power plants, biomass power plants, hydropower plants, solar thermal power plants or combinations thereof.

13. Process according to at least one of the preceding claims, wherein hydrocarbons and/or chemical compounds consisting of carbon, hydrogen and oxygen are generated as the carbon-based secondary energy carriers or base chemicals.

**Revendications**

1. Procédé pour produire des vecteurs d'énergie secondaire à base de carbone (C) ou des substances chimiques de base (8) en couplant une combustion oxygaz (1) de combustibles à base de C (11) et une électrolyse à électrolyte solide à haute température (HT-SOEL) (2, 2A, 2K), dans lequel l'oxygène (3) généré lors de la HT-SOEL est utilisé comme agent oxydant pour la combustion oxygaz, et les gaz de gazogène (4, 4', 5, 5') obtenus lors de la combustion oxygaz et/ou à partir du compartiment cathodique (2K) de la HT-SOEL, ainsi que, le cas échéant, de l'hydrogène (16) fourni en plus depuis l'extérieur, sont convertis de façon exothermique d'une manière connue en soi, en vecteurs d'énergie secondaire à base de C ou en substances chimiques de base (8),
dans lequel, pendant le procédé, de la chaleur est évacuée à différents niveaux de température de telle manière que la chaleur cédée I (6) lors de la synthèse exothermique (7) des vecteurs d'énergie secondaire à base de C ou des substances chimiques de base (8) est utilisée pour générer de la vapeur d'eau pour la HT-SOEL (2, 2A, 2K), la chaleur cédée II inhérente aux gaz de gazogène (3, 4, 4', 5, 5') de la HT-SOEL est utilisée pour préchauffer la vapeur d'eau (9) qui doit être transférée pour la HT-SOEL, et la chaleur cédée III (10) obtenue lors de la combustion oxygaz est fournie à la HT-SOEL (2, 2A, 2K),
dans lequel la HT-SOEL est conduite en mode endothermique, le point de fonctionnement pour la tension de cellule appliquée pour chaque cellule d'électrolyse se situant entre le besoin en énergie électrique deltaG et le besoin total en énergie deltaH, et le manque d'énergie de la HT-SOEL est couvert en mode endothermique par la chaleur cédée III (10),
et dans lequel le rendement électrique lors de la HT- SOEL est supérieur à 100 % et va jusqu'à 140 %, lorsque le rendement électrique ($\eta_{Elektr}$) de la HT- SOEL est défini d'après la Formule (I) suivante :

$$\eta_{Elektr} = ( \ V'_{H2} \ [m^3{}_{STP} / h] * LHV_{H2} \ [kWh / m^3{}_{STP}] ) / P_{el} \ [kW] * 100 \ [\%] \qquad (I)$$

dans laquelle $V'_{H2}$ représente le débit volumique de $H_2$, $LHV_{H2}$ représente le pouvoir calorifique inférieur de l'hydrogène produit, STP représente les conditions normales de température et de pression, et $P_{e1}$ représente la puissance électrique.

2. Procédé selon la revendication 1, dans lequel la combustion oxygaz (1) et la HT-SOEL (2, 2A, 2K) sont réalisées de manière spatialement séparée l'une de l'autre, la chaleur cédée III (10) lors de la combustion oxygaz est indirectement fournie à la HT-SOEL par l'intermédiaire d'au moins un échangeur de chaleur, et un débit massique de dioxyde de carbone (4) obtenu lors de la combustion oxygaz est converti en vecteurs d'énergie secondaire à base de C ou en substances chimiques de base (8), avec l'hydrogène (5) obtenu lors de la HT-SOEL.

3. Procédé selon la revendication 1, dans lequel la combustion oxygaz (1) et la HT-SOEL (2, 2A, 2K) sont réalisées de manière spatialement séparée l'une de l'autre, un gaz de fumée (4') contenant du dioxyde de carbone et de la vapeur d'eau obtenue lors de la combustion oxygaz, et donc la chaleur cédée III obtenue lors de la combustion oxygaz, est directement fourni à la HT-SOEL (2K) sous forme de gaz de conduit cathodique, et un gaz de synthèse (5') obtenu à partir de la HT-SOEL, comprenant de l'hydrogène, du monoxyde de carbone, du dioxyde de carbone et facultativement de la vapeur d'eau, est converti en vecteurs d'énergie secondaire à base de C ou en substances chimiques de base (8).

4. Procédé selon la revendication 1, dans lequel la combustion oxygaz (1) et la HT-SOEL (2, 2A, 2K) sont intégrées spatialement, la HT-SOEL étant réalisée à l'intérieur de la chambre de combustion de la combustion oxygaz, un gaz de fumée (4') contenant du dioxyde de carbone de la vapeur d'eau obtenue lors de la combustion oxygaz, et donc la chaleur cédé III obtenue lors de la combustion oxygaz, est directement fourni à la HT-SOEL (2K) sous forme de gaz de conduit cathodique, et un gaz de synthèse (5') obtenu à partir de la HT-SOEL, comportant de l'hydrogène, du monoxyde de carbone, du dioxyde de carbone et facultativement de la vapeur d'eau, ainsi que de l'oxygène (16) fourni en plus depuis l'extérieur, sont convertis en vecteurs d'énergie secondaire à base de C ou en substances chimiques de base (8).

5. Procédé selon au moins une des revendications précédentes, dans lequel la HT-SOEL (2) est mise en œuvre à des températures de 600 à 1 100 °C, de préférence de 800 à 1 050 °C.

6. Procédé selon au moins une des revendications précédentes, dans lequel la chaleur cédée I (6) a un niveau de température < 500 °C, de préférence d'environ 250 °C.

7. Procédé selon au moins une des revendications précédentes, dans lequel la chaleur cédée II a un niveau de température inférieur à la température de fonctionnement de la HT-SOEL.

8. Procédé selon au moins une des revendications précédentes, dans lequel la chaleur cédée III (10) a un niveau de température supérieur à la température de fonctionnement de la HT-SOEL, généralement de plus de 800 °C, de préférence de plus de 800 à 1 000 °C.

9. Procédé selon au moins une des revendications précédentes, dans lequel le besoin total en oxygène pour la combustion oxygaz (1) est couvert par la HT-SOEL (2).

10. Procédé selon au moins une des revendications précédentes, dans lequel des combustibles biogènes (11) se présentant sous forme de combustibles solides et/ou de combustibles liquides et/ou de gaz combustibles sont utilisés en tant que combustibles à base de C (11), dans lequel les combustibles solides sont du bois de chauffage, des résidus de fermentation ou des eaux résiduaires, dans lequel les combustibles liquides sont des huiles végétales, et dans lequel les gaz combustibles sont des gaz de fermentation ou des gaz de digestion.

11. Procédé selon au moins une des revendications précédentes, dans lequel une production d'électricité à partir de sources renouvelables est utilisée en tant qu'énergie électrique (12) pour la HT-SOEL (2).

12. Procédé selon la revendication 11, dans lequel la production d'électricité à partir de sources renouvelables s'effectue au moyen d'installations éoliennes, d'installations photovoltaïques, de centrales géothermiques, de centrales de biomasse, de centrales hydrauliques, de centrales thermosolaires ou de combinaisons de celles-ci.

13. Procédé selon au moins une des revendications précédentes, dans lequel des hydrocarbures et/ou des composés chimiques constitués de carbone, d'hydrogène et d'azote sont générés en tant que vecteurs d'énergie secondaire à base de C ou substances chimiques de base.

Fig. 1

Fig. 2

EP 3 390 693 B1

Fig. 3

FIG. 4

FIG. 5

Elektrolyse: $CO_2 \rightarrow 1/2\ O_2 + CO$

Zellspannung [Volt]

Temperatur [°C]

$\Delta H$ (gesamter Energiebedarf)

$\Delta G$ (elektrischer Energiebedarf)

$T\Delta S$ (Wärmebedarf)

1,46 V

0,88 V

1300 K

Energiebedarf zur CO-Erzeugung
[kWh/(m³ CO i.N.)]

17

**EP 3 390 693 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010115983 A1
- EP 2360230 A1
- DE 102006035893 A1
- WO 2014167477 A1
- WO 2014170200 A1
- US 20110041740 A